# EUROPÄISCHE PATENTSCHRIFT

(11) **EP 4 216 863 B1**
(45) Veröffentlichungstag und Bekanntmachung des Hinweises auf die Patenterteilung: **03.01.2024**
(21) Anmeldenummer: 21819822.4
(22) Anmeldetag: 25.11.2021
(51) Int. Cl.: A61B 46/10, A61B 90/20

(54) **HALTEELEMENT FÜR EIN DRAPE FÜR EIN OPERATIONSMIKROSKOP**
HOLDING ELEMENT FOR A DRAPE FOR A SURGICAL MICROSCOPE
ÉLÉMENT DE MAINTIEN POUR UN CHAMP OPÉRATOIRE POUR UN MICROSCOPE CHIRURGICAL

(30) Priorität: 27.11.2020 DE 102020131496
(43) Veröffentlichungstag der Anmeldung: 02.08.2023
(73) Patentinhaber: Carl Zeiss Meditec AG, 07745 Jena (DE)
(72) Erfinder: RAAB, Andreas, 73491 Neuler (DE); KÖNIG, Frank, 73431 Aalen (DE)
(74) Vertreter: Tautz & Schuhmacher
(86) Internationale Anmeldenummer: PCT/EP2021/082928
(87) Internationale Veröffentlichungsnummer: WO 2022/112385

(56) Entgegenhaltungen:
- EP-A1- 3 178 438
- DE-A1-102018 107 357
- DE-U1-202004 021 969

## Beschreibung

Die vorliegende Erfindung betrifft ein Halteelement für ein Drape für ein Operationsmikroskop, und ein System mit einem Halteelement und dem Operationsmikroskop.

Herkömmliche Operationsmikroskope weisen in der Regel ein symmetrisches, meist rundes (Haupt-) Objektiv auf, das im Betrieb des Operationsmikroskops abgedeckt sein kann, insbesondere um eine Verschmutzung des Operationsmikroskops und/oder eine Verunreinigung einer zu behandelnden Wunde zu vermeiden.

Zur Abdeckung wird eine sterile Schmutzschutzhülle bzw. ein sog. steriles Drape über ein an dem Drape befestigtes, insbesondere angeschweißtes, Halteelement mit einem Haltering an einem optischen Ein- und/oder Ausgang, insbesondere am Objektiv, des Operationsmikroskops befestigt, anschließend über das Operationsmikroskop gestülpt bzw. gezogen und am Operationsmikroskop beispielsweise über Klebebänder fixiert. Das Drape wird in der Regel vor jeder Operation erneuert und ist damit ein Einwegprodukt.

Die Befestigung am Objektiv, welche beispielsweise durch eine Klemmung erreicht werden kann, muss sicher halten, um ein Herabfallen des Halterings bzw. des Klemmrings mit dem Drape, insbesondere während einer Operation, zu verhindern.

Herkömmlich ist im oder am Haltering ein Objektivschutzglas, das als wechselbares Teil ausgeführt sein kann, montiert. Das Objektivschutzglas wird in den Haltering über Führungen eingeschoben und dort gehalten. Das Objektivschutzglas kann parallel zum Objektiv ausgerichtet sein oder eine definierte Neigung zum Objektiv aufweisen, sodass durch das Objektivschutzglas erzeugte Reflexe verhindert bzw. minimiert werden können. Dem Chirurgen werden so eine im Wesentlichen reflexfreie Sicht und damit ein ergonomisches Arbeiten ermöglicht.

Nachteilig an bekannten Drapes und insbesondere deren Halteringen ist, dass der Haltering bei einer Montage frei auf dem Hauptobjektiv verdreht werden kann und muss, bis die richtige bzw. vorbestimmte Position des Halterings gefunden wurde.

Zudem können neben dem Hauptobjektiv weitere optische Systeme benötigt werden, für welche bei herkömmlichen Drapes zusätzliche, separate Fenster bzw. Aussparungen für weitere Objektivschutzgläser vorgesehen sein müssen.

Weiterhin muss ein Bediener eines herkömmlichen Operationsmikroskops bei der Benutzung desselbigen, wenn er für eine sichere Bedienung des Operationsmikroskops zu starke Reflexe feststellt, einen herkömmlichen Haltering manuell nachpositionieren. Dies kann zum einen zu einer Unsterilität des Drapes und dadurch zu einem notwendigen erneuten Drapen, d.h. zu einem erneuten Abdecken des Operationsmikroskops mit einem weiteren, noch sterilen Drape, führen. Zum anderen kann das Nachpositionieren zu einem Zug auf das Drape und einer damit einhergehenden Beschädigung desselben führen. Dies kann einen Operationsverlauf negativ beeinflussen und sollte daher vermieden werden.

Die Deutsche Patentanmeldung DE 10 2018 107 357 A1 offenbart einen Montageadapter zur lösbaren Befestigung eines Sterilüberzugs an einem Objektiv eines Mikroskops, mit einem Durchgang für das Einführen des Objektivs, wobei in dem Durchgang eine Drapelinse oder Licht-transparente Scheibe angeordnet ist, derart, dass diese einen schiefen Winkel zur Längsachse des Durchgangs einnimmt und mit einer vorzugsweise manuell betätigbaren Rasteinrichtung für das axiale Halten des Montageadapters am in den Durchgang eingeführten Objektiv. Es ist eine Positioniergeometrie vorgesehen, die dafür ausgebildet und angeordnet ist, eine reproduzierbare Soll-Winkelpositionierung oder Soll- Rotationsausrichtung des Montageadapters am Objektiv vorzugsweise ausschließlich durch dessen axiales Aufstecken zu bewirken und weiter vorzugsweise unabhängig und separat zur Rastvorrichtung ihre Positionierfunktion zu erfüllen.

Die Europäische Patentanmeldung EP 3 178 438 A1 offenbart einen Schutzglasadapter für ein Operationsmikroskop, aufweisend eine erste Anlagefläche, wobei die erste Anlagefläche derart ausgebildet ist, dass diese mit einer korrespondierenden zweiten Anlagefläche des Operationsmikroskops in Kontakt bringbar ist. Der Schutzglasadapter weist eine Führungsöffnung zur Aufnahme eines Hauptobjektivs des Operationsmikroskops auf, wobei die Führungsöffnung als Kegelstumpf mit einer ersten Zentralachse ausgebildet ist, wobei der größere Durchmesser des Kegelstumpfes zu der ersten Anlagefläche hin ausgebildet ist. Der Schutzglasadapter weist mindestens ein Positionierungselement auf, wobei das mindestens eine Positionierungselement starr ausgebildet ist und an der ersten Anlagefläche oder an der Oberfläche der Führungsöffnung angeordnet ist, wobei das Positionierungselement zur formschlüssigen Aufnahme in einem Gegenelement an dem Operationsmikroskop geeignet ist. Der Schutzglasadapter weist mindestens ein Halteelement auf, wobei durch das Halteelement eine Haltekraft parallel zu der ersten Zentralachse zwischen dem Schutzglasadapter und dem Operationsmikroskop bewirkbar ist, wobei die Haltekraft durch eine Magnetkraft gebildet ist.

Eine Aufgabe der vorliegenden Erfindung ist es daher, eine Vorrichtung bereitzustellen, die ausgestaltet ist, zumindest einen der oben beschriebenen Nachteile des Standes der Technik zu überwinden.

Insbesondere soll eine Lösung bereitgestellt werden, die eine einfachere, genauere und/oder sicherere Positionierung eines Halteelements eines Drapes an einem Operationsmikroskop ermöglicht und insbesondere eine wechselseitige Beeinflussung verschiedener optischer Systeme des Operationsmikroskops verhindert.

Diese Aufgabe wird erfindungsgemäß durch Vorrichtungen gemäß den unabhängigen Ansprüchen gelöst, wobei vorteilhafte Ausgestaltungen in den abhängigen Ansprüchen angegeben sind.

Demnach wird die Aufgabe durch ein Halteelement für ein Drape für ein Operationsmikroskop gelöst, wobei das Halteelement einen Haltering und eine in dem Haltering angeordnete Ausnehmung für eine Schnittstelle des Operationsmikroskops aufweist.

Der Haltering weist eine die Ausnehmung begrenzende Kontur auf.

Die Kontur ist dabei so ausgebildet, dass in einem Zustand, in dem das Halteelement an dem Operationsmikroskop befestigt ist, eine einzige mögliche Befestigungsposition des Halteelements an der zu der Kontur des Halterings korrespondierenden Schnittstelle des Operationsmikroskops existiert. In der Befestigungsposition ist eine Ausrichtung des Halteelements relativ zum Operationsmikroskop festgelegt.

Um dies zu erreichen zeichnet sich das Halteelement dadurch aus, dass die Kontur in einem Abschnitt der Kontur asymmetrisch ausgebildet ist, wobei der (asymmetrische) Abschnitt durch einen Innenwinkel von zumindest 90° begrenzt ist.

Die Kontur weist vorliegend insgesamt einen Öffnungswinkel von 360° auf. Das heißt, es handelt sich um eine umlaufende Kontur, welche die Ausnehmung vollständig umschließt. In zumindest einem Abschnitt der Kontur, der durch den Innenwinkel definiert bzw. begrenzt ist, ist die Kontur als Ganzes asymmetrisch ausgebildet. Mit anderen Worten ist die Kontur über den ganzen durch den Innenwinkel begrenzten Abschnitt asymmetrisch ausgebildet. Das heißt, in diesem Abschnitt ist nicht lediglich in einem kleinen Bereich eine Asymmetrie, beispielsweise in Form einer Ausnehmung oder Nut vorgesehen, sondern die Kontur als solche, die eine eindimensionale Menge an Punkten entlang eines Außenumfangs der Ausnehmung beschreibt, ist (in sich) asymmetrisch. Denkbar ist, dass die Kontur in dem asymmetrischen Abschnitt vollständig asymmetrisch ausgebildet ist.

Auf diese Weise kann bei der Montage des Halteelements automatisch eine vordefinierte Befestigungsposition des Halteelements an einer zu der Kontur des Halterings korrespondierenden Schnittstelle des Operationsmikroskops erreicht werden. Insbesondere kann auf diese Weise ein Nutzer die nötige Ausrichtung des Halteelements an die Befestigungsposition gut erkennen. Damit kann das Halteelement besonders zeitsparend montiert werden.

Der Innenwinkel kann in einer Ebene, die senkrecht bzgl. einer parallel zur Befestigungsrichtung des Halteelements verlaufenden Achse angeordnet ist, ausgebildet sein.

Beispielsweise kann der asymmetrische Abschnitt durch einen Innenwinkel von zumindest 100°, 110°, 120°, 130°, 140°, 150°, 160°, 170°, 180°, 270° oder 360° begrenzt sein.

Unter "asymmetrisch" kann vorliegend eine die Ausnehmung begrenzende Kontur verstanden werden, welche zumindest in dem durch den Innenwinkel von zumindest 90°, optional von zumindest 110°, 120°, 130°, 140°, 150°, 160°, 170°, 180°, 270° oder 360°, begrenzten Abschnitt nicht rotationssymmetrisch, insbesondere nicht drehsymmetrisch, insbesondere nicht drehsymmetrisch bzgl. der parallel zur Befestigungsrichtung des Halteelements verlaufenden Achse, ausgebildet ist. Denkbar ist jedoch, dass die Kontur in diesem Abschnitt spiegelsymmetrisch ausgebildet ist. Denkbar ist jedoch auch, dass die Kontur in diesem Abschnitt weder rotationssymmetrisch noch spiegelsymmetrisch ausgebildet ist.

Mit anderen Worten, durch die Asymmetrie der die Ausnehmung begrenzenden Kontur, d.h. der Innenkontur des Halterings, zentriert sich das Halteelement zu einer Referenzachse des Operationsmikroskops und ist lateral fixiert. Über eine axiale Anlagefläche am Haltering wird das Halteelement in seiner axialen Lage im Raum positioniert. Die Position des Halteelements ist dadurch in den drei Freiheitsgraden definiert.

Zudem kann der Haltering bei bzw. nach der Montage am Operationsmikroskop nicht mehr frei verdreht werden. Die richtige bzw. gewünschte Position wird automatisch erhalten. Damit kann vermieden werden, dass das Halteelement, beispielsweise aufgrund von bei der Benutzung des Operationsmikroskops festgestellten stärkeren Reflexen, nachpositioniert werden muss. Folglich kann eine durch das Nachpositionieren auftretende Unsterilität des Drapes und ein dadurch notwendiges erneutes Drapen und/oder eine durch das Nachpositionieren auftretende Beschädigung des Drapes, z.B. durch Zug auf das Drape, vermieden werden.

Das oben beschriebene Halteelement vermeidet folglich bei herkömmlichen Halteelementen auftretende Störungen, die einen Ablauf einer Operation negativ beeinflussen können.

Unter einem Drape, das auch als Schmutzschutzhülle bezeichnet werden kann, kann vorliegend eine sterile Einweghülle verstanden werden. Solche Einweghüllen werden beispielsweise in der Chirurgie verwendet, um unsterile Geräte, wie vorliegend das Operationsmikroskop, abzudecken, damit durch die Bedienung des Gerätes für eine steril gekleidete Person keine Gefahr besteht, mit unsterilen Oberflächen in Berührung zu kommen. Das Drape kann durch Schweißen zum Halteelement verbunden sein.

Im bzw. am Haltering kann ein Objektivschutzglas als wechselbares Teil montiert sein. Durch die Ausnehmung und das Obj ektivschutzglas hindurch können optische Ein- und/oder Ausgänge des Operationsmikroskops auf einen Operationsbereich gerichtet werden. Die Ausnehmung kann demnach eine Größe bzw. eine Abmessung eines optischen Fensters festlegen, durch das eine optische Achse einer Optik des Operationsmikroskops verläuft. Die Ausnehmung kann auch als optisches Fenster bezeichnet werden.

Das Merkmal "der asymmetrischen, die Ausnehmung begrenzenden Kontur" kann vorliegend dahingehend verstanden werden, dass der Haltering im Querschnitt, d.h. im Schnitt in einer Ebene, auf der eine Befestigungsrichtung des Halteelements senkrecht steht, in einem Bereich eine asymmetrische Form aufweist, der mit der Schnittstelle des Operationsmikroskops in dem Zustand in Kontakt ist, in dem das Halteelement an dem Operationsmikroskop befestigt ist.

Der Begriff "Ring" bzw. "Haltering" kann vorliegend als ein in sich geschlossener Gegenstand bzw. eine in sich geschlossene Vorrichtung verstanden werden, die eine als Durchgangsloch ausgebildete Ausnehmung für die Schnittstelle des Operationsmikroskops aufweist. Eine äußere Abmessung des Durchgangslochs bzw. der Ausnehmung wird durch die oben beschriebene Kontur begrenzt.

Der Haltering weist eine in der Befestigungsrichtung des Halteelements unter einem vorbestimmten Winkel geweitete bzw. sich weitende Struktur auf. Diese geweitete Struktur kann auch als konisch oder sich verjüngende Struktur bezeichnet werden.

Der Haltering trägt mit seiner oben beschriebenen asymmetrischen Form für den Anwender die eindeutige Information in sich, in welcher Richtung das Halteelement am Operationsmikroskop zu positionieren ist. Des Weiteren hilft die in der Befestigungsrichtung bzw. Aufsatzrichtung unter dem Winkel geweitete Struktur dem Anwender beim leichteren Positionieren des Halteelements.

Der Haltering kann an seiner inneren, der Ausnehmung für die Schnittstelle des Operationsmikroskops zugewandten Oberfläche eine Gummierung aufweisen. Damit kann das Halteelement durch Haftreibung in der einzigen möglichen Befestigungsposition des Halteelements an der zur Kontur des Halterings korrespondierenden Schnittstelle des Operationsmikroskops befestigbar sein.

Mit anderen Worten, der Haltering kann in dem Bereich, der mit der Schnittstelle des Operationsmikroskops in dem Zustand in Kontakt ist, in dem das Halteelement an dem Operationsmikroskop befestigt ist, ein Material aufweisen, dass eine Befestigung des Halteelements an der Schnittstelle des Operationsmikroskops durch Haftreibung erlaubt. Zusätzlich oder alternativ kann das Halteelement ein magnetisches und/oder magnetisierbares Material zur Befestigung des Halteelements an dem Operationsmikroskop aufweisen. Bei dem Material kann es sich beispielsweise um Eisen bzw. um eine Eisen enthaltende Legierung handeln.

Mit anderen Worten, der Haltering kann an der Schnittstelle des Operationsmikroskops durch eine magnetische Kraft fixiert bzw. befestigt sein, die zwischen dem Operationsmikroskop, insbesondere dessen Schnittstelle, und dem Halteelement in dem Zustand wirkt, in dem das Halteelement an dem Operationsmikroskop befestigt ist.

Das heißt, die Befestigung kann über eine Gummierung durch Haftung und/oder über Magnete erfolgen. Die Befestigung über Magnete hat den Vorteil, dass ein einhändiges Positionieren des Halte- bzw. Adapterrings an der Schnittstelle des Operationsmikroskops ohne eine Verwendung von zusätzlichen Komponenten ermöglicht bzw. erleichtert wird.

Der Haltering kann eine Führung aufweisen, in die ein Objektivschutzglas eingeführt und in dem eingeführten Zustand gehalten werden kann.

Die Führung kann mehrere, insbesondere vier, Halteelemente, aufweisen, die so angeordnet und ausgestaltet sind, dass das Objektivschutzglas in der Befestigungsrichtung des Halteelements am Halteelement fixiert bzw. befestigt ist. Zudem kann die Führung ein Element aufweisen, das das Objektivschutzglas in einer Einführrichtung des Objektivschutzglases fixiert. Das Element kann als ein Rastelement ausgeführt sein. Insbesondere kann eine Ausnehmung vorgesehen sein, in die das Rastelement am Objektivschutzglas im eingeführten Zustand des Objektivschutzglases eingreifen kann. Ein Herausfallen des Objektivschutzglases im Betrieb des Operationsmikroskops kann damit vermieden werden.

Denkbar ist, dass die Führung so angeordnet und ausgestaltet ist, dass das Objektivschutzglas aus einer Richtung, die senkrecht zur Befestigungsrichtung angeordnet ist, in den Haltering eingeschoben werden kann. Mit anderen Worten, die Führung kann es einem Benutzer ermöglichen, das Objektivschutzglas seitlich in den Haltering einzuschieben. Denkbar ist auch, dass die Führung einen Anschlag aufweist, sodass das Objektivschutzglas beim Einführen maximal bis zu einem vordefinierten Endpunkt eingeschoben werden kann. Dies erleichtert das Einführen und Positionieren des Obj ektivschutzglases.

Die Führung kann eine asymmetrische Form aufweisen, sodass nur eine einzige mögliche Positionierung des Objektivschutzglases in dem eingeführten Zustand desselbigen in dem Haltering existiert, in der eine Ausrichtung des Objektivschutzglases relativ zum Halteelement festgelegt ist. Dies erleichtert das Einführen des Objektivschutzglases weiter.

"Asymmetrisch" bedeutet vorliegend, dass die Führung nicht rotationssymmetrisch ausgebildet ist. Denkbar ist jedoch, dass diese spiegelsymmetrisch ausgebildet ist. Denkbar ist jedoch auch, dass die Führung weder rotationssymmetrisch noch spiegelsymmetrisch ausgebildet ist.

Das heißt, der Haltering kann einen Einschub aufweisen, in den das Objektivschutzglas bzw. die Schutzscheibe eingeschoben und fixiert werden kann. Das Objektivschutzglas und deren Stellung, insbesondere deren Schiefstellung bzw. Neigung, kann so auf das Operationsmikroskop und insbesondere dessen Beleuchtungs- und/oder Beobachtungsoptik angepasst sein, dass störende bzw. vorbestimmte Reflexe im Operationsmikroskop und/oder in einem Strahlengang einer optionalen Beobachtungsoptik, insbesondere einer Tracking-Kamera, vermieden werden können. Durch die innere Kontur des Halterings mit der Asymmetrie kann sichergestellt werden, dass die Schutzscheibe richtig positioniert ist und sich nicht verdrehen kann.

Das Merkmal "der asymmetrischen Form" der Führung kann vorliegend dahingehend verstanden werden, dass die Führung im Querschnitt, d.h. im Schnitt in einer Ebene, auf der die Befestigungsrichtung des Halteelements senkrecht steht, in einem Bereich eine asymmetrische Form bzw. Kontur aufweist, der mit dem Objektivschutzglas in dem eingeführten Zustand desselbigen in Kontakt ist.

Die asymmetrische, die Ausnehmung begrenzende Kontur des Halterings kann einen Kreisabschnitt aufweisen.

Damit und insbesondere im Zusammenwirken mit der oben beschriebenen Verwendung von Magneten kann der Haltering auch an Schnittstellen an Operationsmikroskopen befestigt werden, die keine asymmetrische Form oder keine als Erhebung ausgebildete Schnittstelle aufweisen.

Denkbar ist, dass der Haltering an einer Oberfläche, die mit einer Oberfläche der Schnittstelle des Operationsmikroskops in dem Zustand in Kontakt ist, in dem das Halteelement an dem Operationsmikroskop befestigt ist, eine Erhöhung bzw. einen Stift aufweist, der zur Fixierung in eine korrespondierende Ausnehmung an der Oberfläche der Schnittstelle des Operationsmikroskops eingreift. Die beiden Oberflächen können jeweils auch als Anlagefläche bezeichnet werden, wobei die Befestigungsrichtung senkrecht zu der jeweiligen Anlagefläche stehen kann.

Die asymmetrische, die Ausnehmung begrenzende Kontur des Halterings, kann eine Nut aufweisen. Die Nut kann auch als eine längliche Vertiefung bezeichnet werden, die sich entlang der Befestigungsrichtung des Halteelements erstreckt. Die Schnittstelle des Operationsmikroskops kann einen zur Nut korrespondierenden Überstand aufweisen, der in dem Zustand in die Nut eingreift, in dem das Halteelement an dem Operationsmikroskop befestigt ist. Dadurch kann eine ggf. weitere formschlüssige Verbindung zur Fixierung des Halteelements an der Schnittstelle des Operationsmikroskops erreicht werden.

Weiterhin wird ein Drape mit einem flexiblen Abschnitt und mit einem oben beschriebenen, zu dem flexiblen Abschnitt verbundenen Halteelement bereitgestellt. Bei dem flexiblen Abschnitt kann es sich um eine Folie, insbesondere eine sterile Folie handeln. Die Folie kann auch als Schmutzschutzhülle bezeichnet werden. Der flexible Abschnitt kann so ausgestaltet sein, dass er mittels des Halterings des Halteelements an einem optischen Ein- und/oder Ausgang eines Operationsmikroskops befestigt werden kann. Denkbar ist, dass der flexible Abschnitt so ausgestaltet ist, dass er nach dem Befestigen über das Operationsmikroskop gestülpt bzw. gezogen und ggf. anschließend am Operationsmikroskop fixiert werden kann.

Das oben mit Bezug zum Halteelement und zum Operationsmikroskop Beschriebene gilt analog auch für das Drape und umgekehrt.

Weiterhin wird ein System aufweisend ein Halteelement für ein Drape und ein Operationsmikroskop bereitgestellt.

Das Halteelement weist einen Haltering und eine in dem Haltering angeordnete Ausnehmung für eine Schnittstelle des Operationsmikroskops auf.

Das Operationsmikroskop weist die Schnittstelle zur Befestigung des Halteelements an dem Operationsmikroskop auf.

Der Haltering weist eine asymmetrische, die Ausnehmung begrenzende Kontur auf.

Die Schnittstelle weist eine asymmetrische, die Schnittstelle nach außen begrenzende Kontur auf, die in einem Zustand, in dem das Halteelement an dem Operationsmikroskop befestigt ist, mit der asymmetrischen, die Ausnehmung begrenzenden Kontur des Halterings in Kontakt ist.

Die asymmetrische, die Ausnehmung begrenzende Kontur des Halterings korrespondiert so zu der asymmetrischen, die Schnittstelle nach außen begrenzenden Kontur der Schnittstelle des Operationsmikroskops, dass in dem Zustand, in dem das Halteelement an dem Operationsmikroskop befestigt ist, eine einzige mögliche Befestigungsposition des Halteelements an der asymmetrischen, die Schnittstelle nach außen begrenzenden Kontur der Schnittstelle des Operationsmikroskops existiert. In der Befestigungsposition ist eine Ausrichtung des Halteelements relativ zum Operationsmikroskop festgelegt.

Das System kann das oben beschriebene Halteelement aufweisen, wobei das Halteelement zu dem Drape verbunden sein kann. Das oben mit Bezug zum Halteelement, zum Drape und zum Operationsmikroskop Beschriebene gilt analog auch für das System mit dem Halteelement und dem Operationsmikroskop. Insbesondere kann das Haltelement an der Schnittstelle des Operationsmikroskops befestigt sein.

Die Schnittstelle des Operationsmikroskops kann zumindest zwei optische Ein- und/oder Ausgänge aufweisen. Die in dem Haltering angeordnete Ausnehmung für die Schnittstelle des Operationsmikroskops kann eine solche Dimensionierung aufweisen, dass die zumindest zwei optischen Ein- und/oder Ausgänge des Operationsmikroskops in dem Zustand, in dem das Halteelement an dem Operationsmikroskop befestigt ist, innerhalb der Ausnehmung angeordnet sind.

Mit anderen Worten, neben einem optischen Ein- und/oder Ausgang, insbesondere für eine Hauptoptik des Operationsmikroskops, kann es erforderlich sein, dass das Operationsmikroskop einen weiteren bzw. zusätzlichen optischen Ein- und/oder Ausgang aufweist. Um eine Wechselwirkung bzw. eine gegenseitige Beeinflussung der einzelnen optischen Ein- und/oder Ausgänge zu vermeiden, kann es weiterhin erforderlich sein, diese getrennt voneinander jedoch möglichst nahe an einer optischen Achse der Hauptoptik zu platzieren.

Bei der Hauptoptik kann es sich um ein Objektiv mit fester oder veränderbarer Brennweite handeln. Denkbar ist insbesondere eine bewegliche verfahrbare Optik.

Dadurch, dass die in dem Haltering angeordnete Ausnehmung für die Schnittstelle des Operationsmikroskops eine solche Dimensionierung aufweisen kann, dass die zumindest zwei optischen Ein- und/oder Ausgänge des Operationsmikroskops in dem Zustand innerhalb der Ausnehmung angeordnet sind, in dem das Halteelement an dem Operationsmikroskop befestigt ist, kann für den einzelnen oder die mehreren optischen Ein- und/oder Ausgänge am Operationsmikroskop ein gemeinsames optisches Fenster gebildet werden. Die optischen Achsen mehrerer oder aller optischen Ein- und/oder Ausgänge können folglich durch das von der Ausnehmung definierte optische Fenster verlaufen.

Das heißt, es kann vorliegend ausreichend sein, wenn für die zumindest zwei optischen Ein- und/oder Ausgänge ein einziges Objektivschutzglas vorgesehen ist. Damit kann verhindert werden, dass sich die optischen Ein- und/oder Ausgänge gegenseitig durch verschiedene Winkel oder Positionierungen der Objektivschutzgläser durch Reflexe beeinflussen, wie dies bei herkömmlichen Systemen der Fall sein kann. Des Weiteren kann eine Fehlpositionierung, die bei herkömmlichen Systemen mit mehreren Einzelfenstern auftreten können, vermieden werden.

Denkbar ist, dass einer der zumindest zwei optischen Ein- und/oder Ausgänge für eine (Haupt-) Optik des Operationsmikroskops vorgesehen ist, und der zweite der zumindest zwei optischen Ein- und/oder Ausgänge für ein Beleuchtungselement, das insbesondere ausgestaltet ist, Licht in einer vorbestimmten Wellenlänge zu emittieren, ein Kamerasystem und/oder ein System zum Einblenden von zumindest einer Information in ein Operationsfeld vorgesehen ist.

Mit anderen Worten, der zusätzliche bzw. die zusätzlichen optischen Ein- und/oder Ausgänge kann/können vorgesehen sein, um weitere Anwendungen am Operationsmikroskop neben der Hauptoptik zu ermöglichen, wie zum Beispiel die Beleuchtung in einer speziellen Wellenlänge, die in Verbindung mit Filtern zur Visualisierung benötigt werden kann, das Bereitstellen eines Kamerasystems und/oder die Einblendung einer Struktur in das Operationsfeld bzw. den Operationsbereich.

Das System kann ein Objektivschutzglas, insbesondere mit einer Beschichtung, aufweisen. Bei der Beschichtung kann es sich um eine Antireflexbeschichtung handeln, die ausgestaltet sein kann, um eine Reflexion an dem Objektivschutzglas zu unterdrücken und optional dessen Transmission zu erhöhen.

Der Haltering kann, wie oben beschrieben, eine Führung aufweisen, in die das Objektivschutzglas eingeführt und in eingeführtem Zustand gehalten werden kann.

Diese Führung des Halterings kann eine asymmetrische Form aufweisen, sodass nur eine einzige mögliche Positionierung des Objektivschutzglases in dem eingeführten Zustand desselbigen in dem Haltering existiert, in der eine Ausrichtung des Objektivschutzglases relativ zum Halteelement festgelegt ist.

Die Führung des Halterings und/oder das Objektivschutzglas können so ausgebildet sein, dass das Objektivschutzglas in dem eingeführten Zustand desselbigen in dem Halteelement parallel oder um einen vorbestimmten Winkel geneigt zur Schnittstelle des Operationsmikroskops angeordnet ist.

Dadurch kann eine Ausrichtung des Objektivschutzglases so an das Operationsmikroskop angepasst sein, dass etwaig auftretende, für einen Benutzer des Operationsmikroskops störende Reflexe minimiert werden können.

Nachfolgend wird eine Ausführungsform mit Bezug zu Figuren 1 bis 4 beschrieben.

Es zeigen:
- Figur 1: eine perspektivische Ansicht einer einem Operationsmikroskop abgewandten Seite eines Halteelements gemäß der Ausführungsform.
- Figur 2: eine perspektivische Ansicht einer dem Operationsmikroskop zugewandten Seite des Halteelements aus Figur 1.
- Figur 3: eine perspektivische Ansicht einer für das Halteelement aus Figuren 1 und 2 vorgesehenen Schnittstelle des Operationsmikroskops gemäß der Ausführungsform.
- Figur 4: eine perspektivische Ansicht des an der in Figur 3 gezeigten Schnittstelle montierten Halteelements, das in Figuren 1 und 2 dargestellt ist.

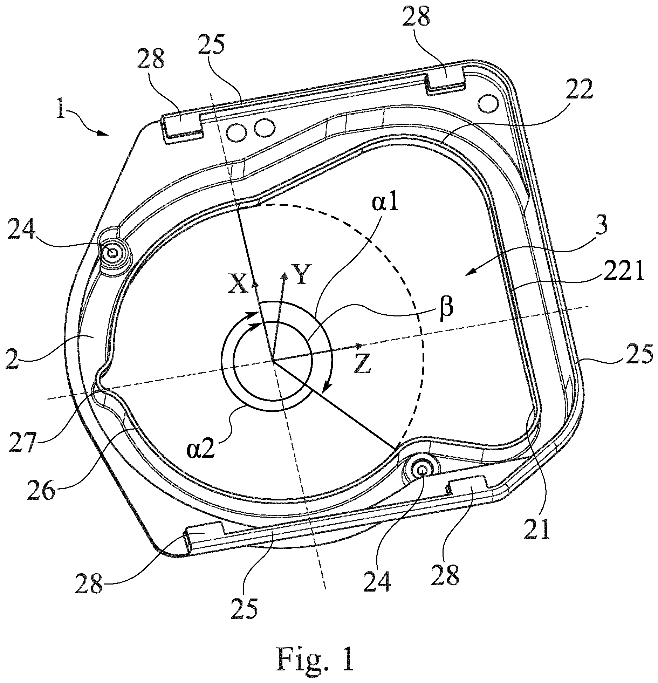

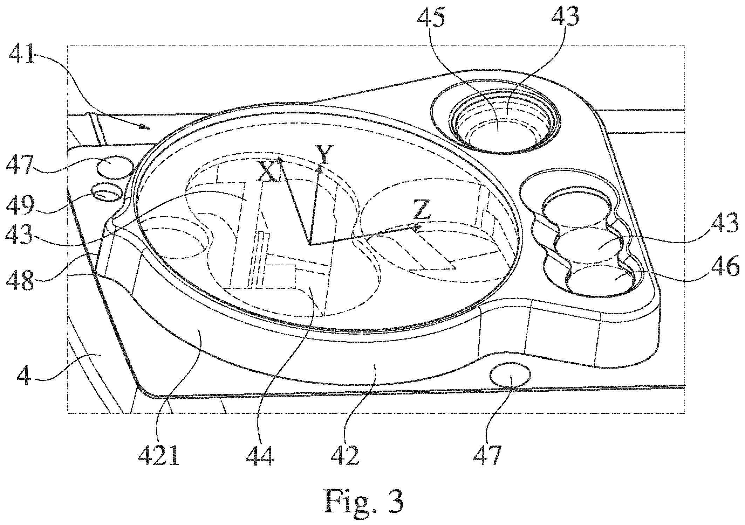

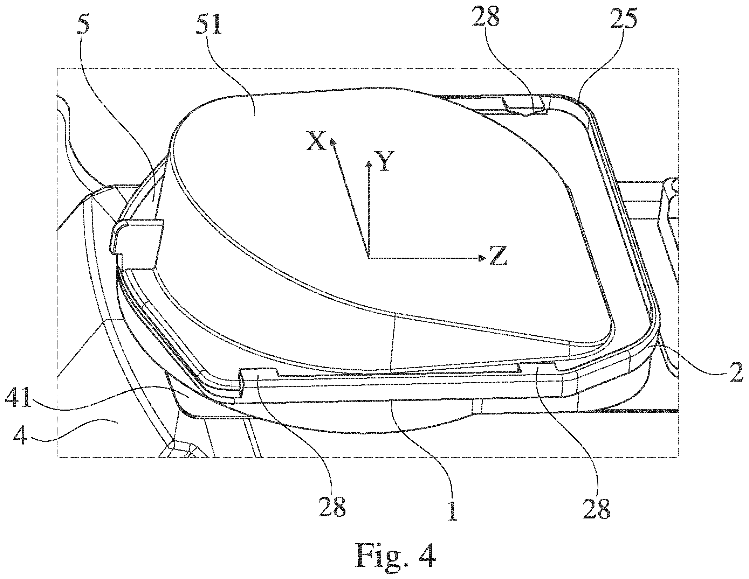

In Figur 1 ist ein Halteelement 1 für ein (nicht dargestelltes) Drape für ein Operationsmikroskop 4 (s. Figuren 3 und 4) gezeigt.

Die in Figur 1 dargestellte Seite des Halteelements 1 ist die Seite, die in einem Zustand, in dem das Halteelement 1 an dem Operationsmikroskop 4 befestigt ist, von dem Operationsmikroskop 4 abgewandt und einem Operationsbereich zugewandt ist. Diese Seite des Halteelements 1 wird nachfolgend als Vorderseite bezeichnet.

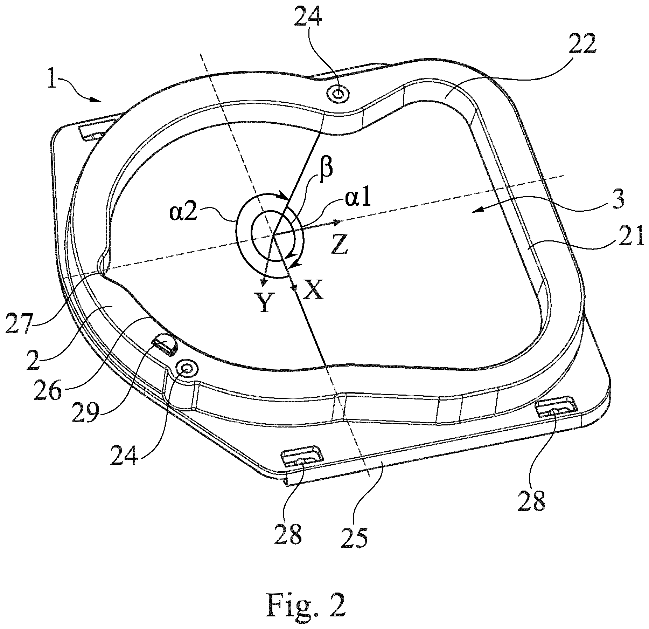

In Figur 2 ist ebenfalls das Halteelement 1 gezeigt. Die in Figur 2 dargestellte Seite des Halteelements 1 ist die Seite, die in einem Zustand, in dem das Halteelement 1 an dem Operationsmikroskop 4 befestigt ist, dem Operationsmikroskop 4 zugewandt und mit einer Schnittstelle 41 des Operationsmikroskops 4 in Kontakt ist (s. auch Figuren 3 und 4).

Diese Seite des Halteelements 1 bildet folglich eine Anlagefläche an der Schnittstelle 41 des Operationsmikroskops 4 und wird nachfolgend als Rückseite bezeichnet.

Ferner ist in Figuren 1 und 2 ein kartesisches Koordinatensystem dargestellt, wobei die Y-Achse des Koordinatensystems einer Montage bzw. Befestigungsrichtung des Halteelements 1 an der Schnittstelle 41 des Operationsmikroskops 4 entspricht. Die Rückseite liegt der Vorderseite in der Befestigungsrichtung Y des Halteelements 1 gegenüber.

Das Halteelement 1 weist einen Haltering 2 und eine in dem Haltering 2 angeordnete Ausnehmung 3 für die Schnittstelle 41 des Operationsmikroskops 4 auf (s. auch Figur 3). Die Ausnehmung 3 ist als Durchgangsloch ausgeführt und erstreckt sich von der Vorder- zur Rückseite des Halteelements 1.

In Figur 3 ist die Schnittstelle 41 des Operationsmikroskops 4 dargestellt. Ferner ist in Figur 3, ebenso wie in Figuren 1 und 2, das kartesische Koordinatensystem dargestellt, wobei auch hier die Y-Achse des Koordinatensystems der Montage bzw. Befestigungsrichtung des Halteelements 1 an der Schnittstelle 41 des Operationsmikroskops 4 entspricht.

Zur Montage bzw. Befestigung des Halteelements 1 wird dieses entlang der Befestigungsrichtung Y auf die Schnittstelle 41 geschoben, sodass eine die Schnittstelle 41 in der X-Richtung und Z-Richtung nach außen begrenzende Oberfläche 421, die eine asymmetrische Kontur 42 aufweist, mit einer die Ausnehmung 3 des Halteelements 1 in der X-Richtung und Z-Richtung nach außen begrenzenden Oberfläche 22 des Halterings 2, die ebenfalls eine asymmetrischen Kontur 21 aufweist, in Kontakt ist. Diese beiden Oberflächen 22, 421 bilden nach der Montage einen Formschluss.

Die Kontur 21 umschließt die in dem Haltering 2 ausgebildete Ausnehmung 3 vollständig und weist daher einen Öffnungswinkel β von 360° in Figur lauf, der vorliegend in einer durch die X-Richtung bzw. X-Achse und die Z-Richtung bzw. Z-Achse aufgespannten Ebene, auf der die Befestigungsrichtung Y senkrecht steht, angeordnet ist.

Die Kontur 21 lässt sich im Wesentlichen in zwei Abschnitte mit einem Innenwinkel α1 von im Wesentlichen 140° und einem Innenwinkel α2 von im Wesentlichen 220° unterteilen, die in Summe dem Öffnungswinkel β von 360° entsprechen, wobei die Innenwinkel α1, α2 jeweils in der durch die X-Achse und die Z-Achse aufgespannten Ebene ausgebildet sind.

In dem Abschnitt, der durch den Innenwinkel α1 aufgespannt bzw. begrenzt wird und damit einen zusammenhängenden Abschnitt von insgesamt im Wesentlichen 140° bildet, ist die Kontur 21 als Ganzes asymmetrisch ausgebildet. Das heißt, die Kontur 21 weist zwar in dem durch den Innenwinkel α1 aufgespannten Bereich einzelne Teilabschnitte auf, die spiegelsymmetrisch und/oder rotationssymmetrisch sind (z.B. der in Figur 1 rechts angeordnete, gerade verlaufende Abschnitt 211 der Kontur 21 ist zumindest teilweise bezüglicher der Z-Achse achsensymmetrisch), jedoch ist die Kontur 21 als Ganzes in dem durch den Innenwinkel α1 aufgespannten Bereich nicht symmetrisch. Die Asymmetrie als Ganzes bezieht sich damit vorliegend auf den gesamten Verlauf der Kontur 21 in dem durch den Innenwinkel α1 aufgespannten Bereich, wobei dieser gesamte Verlauf eine mangelnde Rotationssymmetrie bezüglich jeder parallel zur Befestigungsrichtung Y verlaufenden Achse und hier auch eine mangelnde Achsensymmetrie bezüglich jeder Achse, die vollständig in der durch die X-Achse und die Y-Achse aufgespannten Ebene angeordnet ist, aufweist.

In dem weiteren durch den Innenwinkel α2 aufgespannten Abschnitt der Kontur 21 ist die Kontur 21 durch einen Kreisabschnitt 26 (eines mit gestrichelter Linie angedeuteten Kreises in Figur 1) im Wesentlichen kreisförmig und damit bezüglich der Y-Achse im Wesentlichen rotationssymmetrisch ausgebildet. Der weitere durch den Innenwinkel α2 aufgespannte Abschnitt der Kontur 21 ist damit als Ganzes im Wesentlichen (rotations-) symmetrisch. Dem steht nicht entgegen, dass die Kontur 21 vorliegend in einem kleinen Bereich eine Asymmetrie bzw. eine asymmetrische Stelle in Form einer Nut 27 aufweist. Denkbar ist, dass die asymmetrische Stelle, hier die Nut 27, in einem Winkelbereich von maximal 30°, bevorzugt zwischen 20° und 30°, weiter bevorzugt von 26°, ausgebildet ist.

Durch die asymmetrische Ausgestaltung von sowohl der äußeren Oberfläche 421 der Schnittstelle 41 als auch der inneren Oberfläche 22 des Halterings 2, die, wie oben beschrieben, jeweils in dem Bereich, der nach der Montage in Kontakt mit dem jeweiligen anderen Bauteil ist, die - insbesondere in dem Abschnitt α1 asymmetrische - Kontur 21, 42 aufweisen, wird bei der Montage des Halteelements 1 automatisch eine vordefinierte Befestigungsposition des Halteelements 1 an dem Operationsmikroskop 4 erreicht. Insbesondere dadurch, dass die Kontur 21 in einem Bereich von zumindest 90°, vorliegend im Wesentlichen 140°, als Ganzes asymmetrisch ausgebildet ist, wird bei der Montage die vordefinierte Befestigungsposition des Halteelements 1 an dem Operationsmikroskop 4 in einer ergonomisch einfachen Weise sichergestellt, welche durch die alleinige Ausbildung einer asymmetrischen Stelle, wie der Nut 27, nicht erreicht werden könnte.

Das heißt, in dem Zustand, in dem das Halteelement 1 an dem Operationsmikroskop 4 befestigt ist, existiert nur eine einzige mögliche Befestigungsposition des Halteelements 1 an der zu der Kontur 21 des Halterings 2 korrespondierenden Schnittstelle 41 des Operationsmikroskops 4. In dieser Befestigungsposition ist eine Ausrichtung des Halteelements 1 relativ zum Operationsmikroskop 4 räumlich festgelegt.

Dies erleichtert es einem Bediener des Operationsmikroskops 4 das Halteelement 1 in einer relativ kurzen Zeitspanne richtig zu montieren.

Um den durch die Ausgestaltung des Halteelements 1 und der Schnittstelle 41 des Operationsmikroskops 4 erreichten Effekt der erleichterten und damit ergonomischeren Montage des Halteelements 1 weiter zu verstärken, weist der Haltering 2 eine sich in der Befestigungsrichtung Y des Halteelements 1 unter einem vorbestimmten Winkel geweitete bzw. konische Struktur 22 auf. Die in Figur 3 dargestellte Schnittstelle 41 des Operationsmikroskops 4 weist dafür ebenfalls eine unter einem vorbestimmten Winkel geweitete Struktur 421 auf. Diese an der Schnittstelle 41 ausgebildete Struktur 421 kann auch als Einführschräge bezeichnet werden und korrespondiert zu der unter dem vorbestimmten Winkel geweiteten Struktur 22 des Halterings 2.

Zum Montieren bzw. Befestigen des Halteelements 1 an der Schnittstelle 41 des Operationsmikroskops 4, wird das Halteelement 1 auf der Schnittstelle 41 aufgesetzt und entlang der Befestigungsrichtung Y auf die Schnittstelle 41 aufgeschoben, sodass die jeweiligen geweiteten Strukturen 22, 421 miteinander in Kontakt sind. Damit kann eine geführte Montage des Halteelements 1 an dem Operationsmikroskop 4, insbesondere bis zum Erreichen der Befestigungsposition des Halteelements 1, realisiert werden.

Um eine erleichterte Positionierung des Halteelements 1 bei der Montage desselbigen auch an (nicht dargestellten) Schnittstellen zu ermöglichen, die eine im Wesentlichen kreisförmige und keine asymmetrische Kontur aufweisen, weist die asymmetrische die Ausnehmung 3 begrenzende Kontur 21 des Halterings 2 einen Kreisabschnitt 26 zur Zentrierung des Halteelements 1 auf. Zudem ist eine Erhöhung 29 an der Anlagefläche bzw. der Rückseite des Halterings 2 ausgebildet, die in dem Zustand, in dem das Halteelement 1 an der Schnittstelle 41 montiert ist, in eine korrespondierende Vertiefung 49 an der Schnittstelle 41 eingreift, um das Halteelement 1 gegen eine Verdrehung relativ zur Schnittstelle 41 zu sichern (s. insbesondere Figuren 2 und 3).

Wie oben beschrieben weist der Haltering 2 in dem Kreisabschnitt 26 die Nut 27 auf. In diese Nut 27 kann ein hervorstehender Abschnitt 48 der Schnittstelle 41 eingreifen.

Die Endstellung bzw. Befestigungsposition mit dem auf der Schnittstelle 41 befestigten bzw. montierten Halteelement 1 ist in Figur 4 dargestellt. In dieser Position sind die Schnittstelle 41 und der Haltering 2 formschlüssig zueinander verbunden.

Genauer gesagt, ist in Figur 4 die Schnittstelle 41 des Operationsmikroskops 4 mit dem darauf montierten und befestigten Halteelement 1 dargestellt. Ferner ist in Figur 4, ebenso wie in Figuren 1, 2 und 3, das kartesische Koordinatensystem dargestellt, wobei auch hier die Y-Achse des Koordinatensystems der Montage bzw. Befestigungsrichtung des Halteelements 1 an der Schnittstelle 41 des Operationsmikroskops 4 entspricht.

Damit das Halteelement 1 an der Schnittstelle 41 des Operationsmikroskops 4 gehalten werden kann, sind zwei Ausgestaltungen des Halteelements 1 und der Schnittstelle 41 denkbar, die alternativ oder in Kombination vorgesehen werden können.

Demnach kann der Haltering 2 an seiner inneren, der Ausnehmung 3 für die Schnittstelle 41 des Operationsmikroskops 4 zugewandten Oberfläche 22 eine Gummierung aufweisen. Diese Gummierung ist zumindest in dem Bereich des Halterings 2 vorgesehen, der mit der Schnittstelle 41 in der Befestigungsposition in Kontakt ist, d.h. insbesondere in dem Bereich, in dem die geweitete Struktur 22 am Haltering 2 ausgebildet ist. Dieser Bereich schließt vorliegend an die Rückseite des Halteelements 1 an.

Durch die Gummierung kann das Halteelement 1 durch Haftreibung, erzeugt durch eine Klemmkraft zwischen dem Haltering 2 und der Schnittstelle 41, in der einzigen möglichen Befestigungsposition des Halteelements 1 an der zur Kontur 21 des Halterings 2 korrespondierenden Schnittstelle 41 des Operationsmikroskops 4 befestigt und dort gehalten werden.

Diese Ausgestaltung ist auch umgekehrt denkbar, d.h. zusätzlich oder alternativ kann auch die Schnittstelle 41 eine Gummierung in dem Bereich 421 aufweisen, der mit dem Haltering 2 in der Befestigungsposition desselbigen in Kontakt ist.

Zusätzlich oder alternativ zur Gummierung kann das Halteelement 1 ein magnetisches Material 24 zur Befestigung des Halteelements 1 an der Schnittstelle 41 des Operationsmikroskops 4 aufweisen.

Dabei ist denkbar, dass an dem Haltering 2 an mehreren Stellen, vorliegend an zwei Stellen (s. Figur 1), ein magnetisches Material 24 angeordnet ist. An zu der Stelle bzw. den Stellen, an der/denen das magnetische Material 24 am Haltering 2 angeordnet ist, korrespondierenden Stellen an der Schnittstelle 41 kann ebenfalls ein magnetisches Material 47 angeordnet sein. Durch zwischen den magnetischen Materialien 24, 47 wirkende magnetische Kräfte kann das Halteelement 1 an der Schnittstelle 41 des Operationsmikroskops 4 befestigt und dort gehalten werden.

Zudem ist in Figur 4 das in eine am Haltering 2 des Halteelements 1 angebrachte Führung 25 eingeschobene Objektivschutzglas 5 dargestellt. Dieses wird durch die Führung 25 in der Befestigungsrichtung Y gehalten, sodass ein Herausfallen des Objektivschutzglases 5 in Richtung des Operationsbereichs vermieden wird. Dazu weist die Führung 25 zumindest ein Halteelement, vorliegend vier an der Führung 25 angeordnete Halteelemente 28 für das Objektivschutzglas 5 auf (s. insbesondere Figur 1), die das Objektivschutzglas 5 in dem eingeführten Zustand halten.

Zum Einführen des Objektivschutzglases 5 wird dieses, insbesondere nach der Montage des Halteelements 1 an der Schnittstelle 41, seitlich, hier entlang der Z-Richtung, in die Führung 25 eingeschoben. Die Führung 25 weist in der Einführrichtung Z des Objektivschutzglases 5 einen (End-) Anschlag auf, sodass eine Endstellung des Objektivschutzglases 5 in der Einführrichtung Z festgelegt ist.

Weiterhin weisen die Halte- bzw. Führungselemente 28 jeweils integrierte Rastelemente für das Objektivschutzglas 5 auf (s. insbesondere Figur 2), sodass im Betrieb des Operationsmikroskops 4 ein Herausfallen bzw. ein Herausgleiten des Objektivschutzglases 5 entgegen der Einführrichtung Z verhindert wird. Die Führungselemente 28 mit integrierter Rastung ermöglichen neben der Fixierung des Objektivschutzglases 5 in dessen Endposition eine spielfreie Führung des Objektivschutzglases 5.

Je zwei der vier Halteelemente 28 bilden zusammen mit einer linken bzw. rechten (d.h. sich an der X-Achse gegenüberliegenden) Seitenfläche der Führung 25 und einer Auflagefläche an dem Haltering 2, auf der die Befestigungsrichtung Y senkrecht steht, eine Längsnut zur Führung des Objektivschutzglases 5.

Ebenso wie die Schnittstelle 41 und der Haltering 2 weist die Führung 25 und auch das Objektivschutzglas 5 eine asymmetrische Form auf, sodass nur eine einzige mögliche Positionierung des Objektivschutzglases 5 in dem eingeführten Zustand desselbigen in dem Haltering 2 existiert. In dem eingeführten Zustand des Objektivschutzglases 5 ist eine Ausrichtung des Objektivschutzglases 5 relativ zum Haltering 2 festgelegt. Damit ist auch eine Ausrichtung des Objektivschutzglases 5 relativ zum Operationsmikroskop 4 festgelegt.

Die Schnittstelle 41 des Operationsmikroskops 4 weist vorliegend drei optische Ein- und/oder Ausgänge 43 auf. Einer der drei optischen Ein- und/oder Ausgänge 43 ist für eine (Haupt-) Optik 44 des Operationsmikroskops 4 vorgesehen. Die zwei weiteren der drei optischen Ein- und/oder Ausgänge 43 sind für ein Beleuchtungselement 46, das insbesondere ausgestaltet ist, Licht in einer vorbestimmten Wellenlänge zu emittieren, und ein Kamerasystem 45 vorgesehen. Denkbar wäre ein vierter optischer Ein- und/oder Ausgang 43, der für ein System zum Einblenden von zumindest einer Information in ein Operationsfeld vorgesehen ist.

Die in dem Haltering 2 angeordnete Ausnehmung 3 für die Schnittstelle 41 des Operationsmikroskops 4 weist eine solche Dimensionierung auf, dass die drei optischen Ein- und/oder Ausgänge 43 des Operationsmikroskops 4 in dem Zustand innerhalb der Ausnehmung 3 angeordnet sind, in dem das Halteelement 1 an dem Operationsmikroskop 4 befestigt ist.

Es ist daher ausreichend, für die drei optischen Ein- und/oder Ausgänge 43 ein einziges Objektivschutzglas 5 vorzusehen.

Damit kann unter anderem verhindert werden, dass sich die optischen Ein- und/oder Ausgänge 43 gegenseitig durch verschiedene Winkel oder Positionierungen der Objektivschutzgläser durch Reflexe beeinflussen. Des Weiteren kann eine Fehlpositionierung, die bei herkömmlichen Systemen mit mehreren Einzelfenstern auftreten kann, vermieden werden.

Wie Figur 4 zu entnehmen ist, ist das Objektivschutzglas 5 vorliegend so ausgebildet, dass eine dem Operationsfeld zugewandte Oberfläche 51 des Objektivschutzglases 5 in dem eingeführten Zustand desselbigen in dem Halteelement 1 um einen vorbestimmten Winkel geneigt zur Schnittstelle 41 des Operationsmikroskops 4 angeordnet ist.

Denkbar wäre auch, dass die Neigung des Objektivschutzglases 5 durch eine entsprechende konstruktive Ausgestaltung der Führung 25 erzeugt wird.

Durch Vorsehen der Neigung kann der oben beschriebene Vorteil der Vermeidung von Reflexen weiter verstärkt werden. Denkbar ist auch, dass das Objektivschutzglas 5 und insbesondere dessen dem Operationsfeld zugewandte Oberfläche 51 zu diesem Zweck eine Antireflexbeschichtung aufweist.

### Bezugszeichenliste

- 1: Halteelement für Drape
- 2: Haltering
- 21: asymmetrische, die Ausnehmung begrenzende Kontur
- 211: achsensymmetrischer Abschnitt
- 22: Oberfläche am Haltering mit in Befestigungsrichtung geweiteter Struktur mit Gummierung
- 24: magnetisches Material
- 25: Führung für Objektivschutzglas mit Endanschlag in Einführrichtung
- 26: Kreisabschnitt der Kontur
- 27: Nut in der die Ausnehmung begrenzenden Kontur
- 28: Halteelement für Objektivschutzglas
- 29: Erhöhung bzw. hervorstehender Abschnitt am Haltering
- 3: Ausnehmung für Schnittstelle des Operationsmikroskops
- 4: Operationsmikroskop
- 41: Schnittstelle für Halteelement
- 42: asymmetrische, die Schnittstelle nach außen begrenzende Kontur
- 421: Oberfläche an Schnittstelle mit in Befestigungsrichtung geweiteter Struktur
- 43: optische Ein- und/oder Ausgänge des Operationsmikroskops
- 44: (Haupt-) Optik des Operationsmikroskops
- 45: Kamerasystem
- 46: Beleuchtungselement
- 47: magnetisches Material
- 48: hervorstehender Abschnitt an Schnittstelle für Nut am Haltering
- 49: zur Erhöhung am Haltering korrespondierende Vertiefung an Schnittstelle
- 5: (wechselbares) Objektivschutzglas
- 51: dem Operationsfeld zugewandte Oberfläche des Objektivschutzglases
- α1, α2: Innenwinkel der die Ausnehmung begrenzenden Kontur
- β: Öffnungswinkel der die Ausnehmung begrenzenden Kontur

## Patentansprüche

1. Halteelement (1) für ein Drape für ein Operationsmikroskop (4),
wobei das Halteelement (1) einen Haltering (2) und eine in dem Haltering (2) angeordnete Ausnehmung (3) für eine Schnittstelle (41) des Operationsmikroskops (4) aufweist, und
wobei der Haltering (2) eine die Ausnehmung (3) begrenzende Kontur (21) aufweist,
**dadurch gekennzeichnet, dass**
die Kontur (21) in zumindest einem Abschnitt der Kontur (21) nicht drehsymmetrisch bezüglich einer parallel zu einer Befestigungsrichtung (Y) des Halteelements (1) verlaufenden Achse ausgebildet ist, wobei der Abschnitt durch einen Innenwinkel (α1) von zumindest 90° begrenzt ist, sodass in einem Zustand, in dem das Halteelement (1) an dem Operationsmikroskop (4) befestigt ist, eine einzige mögliche Befestigungsposition des Halteelements (1) an der zu der Kontur (21) des Halterings (2) korrespondierenden Schnittstelle (41) des Operationsmikroskops (4) existiert, in der eine Ausrichtung des Halteelements (1) relativ zum Operationsmikroskop (4) festgelegt ist, und
der Haltering (2) eine in der Befestigungsrichtung (Y) des Halteelements (1) unter einem vorbestimmten Winkel geweitete Struktur (22) aufweist.

2. Halteelement (1) gemäß Anspruch 1, **dadurch gekennzeichnet, dass** der Haltering (2) an seiner inneren, der Ausnehmung (3) für die Schnittstelle (41) des Operationsmikroskops (4) zugewandten Oberfläche (22) eine Gummierung aufweist, sodass das Halteelement (1) durch Haftreibung in der einzigen möglichen Befestigungsposition des Halteelements (1) an der zur Kontur (21) des Halterings (2) korrespondierenden Schnittstelle (41) des Operationsmikroskops (4) befestigbar ist.

3. Halteelement (1) gemäß Anspruch 1 oder 2, **dadurch gekennzeichnet, dass** das Halteelement (1) ein magnetisches und/oder magnetisierbares Material (24) zur Befestigung des Halteelements (1) an dem Operationsmikroskop (4) aufweist.

4. Halteelement (1) gemäß einem der vorangehenden Ansprüche, **dadurch gekennzeichnet, dass** der Haltering (2) eine Führung (25) aufweist, in die ein Objektivschutzglas (5) eingeführt und in eingeführtem Zustand gehalten werden kann.

5. Halteelement (1) gemäß Anspruch 4, **dadurch gekennzeichnet, dass** die Führung (25) eine asymmetrische Form aufweist, sodass nur eine einzige mögliche Positionierung des Objektivschutzglases (5) in dem eingeführten Zustand desselbigen in dem Haltering (2) existiert, in der eine Ausrichtung des Objektivschutzglases (5) relativ zum Halteelement (1) festgelegt ist.

6. Halteelement (1) gemäß einem der vorangehenden Ansprüche, **dadurch gekennzeichnet, dass** die asymmetrische, die Ausnehmung (3) begrenzende Kontur (21) des Halterings (2) zumindest einen Kreisabschnitt (26) aufweist.

7. Halteelement (1) gemäß einem der vorangehenden Ansprüche, **dadurch gekennzeichnet, dass** die asymmetrische, die Ausnehmung (3) begrenzende Kontur (21) des Halterings (2) eine Nut (27) aufweist.

8. Drape mit einem flexiblen Abschnitt und einem zu dem flexiblen Abschnitt verbundenen Halteelement (1) nach einem der vorangehenden Ansprüche.

9. System aufweisend ein Halteelement (1) für ein Drape und ein Operationsmikroskop (4), wobei
das Halteelement (1) einen Haltering (2) und eine in dem Haltering (2) angeordnete Ausnehmung (3) für eine Schnittstelle (41) des Operationsmikroskops (4) aufweist, und
das Operationsmikroskop (4) die Schnittstelle (41) zur Befestigung des Halteelements (1) an dem Operationsmikroskop (4) aufweist,
**dadurch gekennzeichnet, dass**
der Haltering (2) eine asymmetrische, die Ausnehmung (3) begrenzende Kontur (21) aufweist, und
die Schnittstelle (41) eine asymmetrische, die Schnittstelle (41) nach außen begrenzende Kontur (42) aufweist, die in einem Zustand, in dem das Halteelement (1) an dem Operationsmikroskop (4) befestigt ist, mit der asymmetrischen, die Ausnehmung (3) begrenzende Kontur (21) des Halterings (2) in Kontakt ist,
wobei die asymmetrische, die Ausnehmung (3) begrenzende Kontur (21) so zu der asymmetrischen, die Schnittstelle (41) nach außen begrenzenden Kontur (42) korrespondiert, dass in dem Zustand, in dem das Halteelement (1) an dem Operationsmikroskop (4) befestigt ist, eine einzige mögliche Befestigungsposition des Halteelements (1) an der Schnittstelle (41) existiert, in der eine Ausrichtung des Halteelements (1) relativ zum Operationsmikroskop (4) festgelegt ist.

10. System gemäß Anspruch 9, **dadurch gekennzeichnet, dass** das System das Halteelement (1) gemäß einem der Ansprüche 1 bis 7 aufweist, wobei das Halteelement (1) optional zu einem flexiblen Abschnitt verbunden ist.

11. System gemäß Anspruch 9 oder 10, **dadurch gekennzeichnet, dass**
die Schnittstelle (41) des Operationsmikroskops (4) zumindest zwei optische Ein- und/oder Ausgänge (43) aufweist, und
die in dem Haltering (2) angeordnete Ausnehmung (3) für die Schnittstelle (41) des Operationsmikroskops (4) eine solche Dimensionierung aufweist, dass die zumindest zwei optischen Ein- und/oder Ausgänge (43) des Operationsmikroskops (4) in dem Zustand innerhalb der Ausnehmung (3) angeordnet sind, in dem das Halteelement (1) an dem Operationsmikroskop (4) befestigt ist.

12. System gemäß Anspruch 11, **dadurch gekennzeichnet, dass**
einer der zumindest zwei optischen Ein- und/oder Ausgänge (43) für eine Optik (44) des Operationsmikroskops (4) vorgesehen ist, und
der zweite der zumindest zwei optischen Ein- und/oder Ausgänge (43) für ein Beleuchtungselement (46), das optional ausgestaltet ist, Licht in einer vorbestimmten Wellenlänge zu emittieren, ein Kamerasystem (45) und/oder ein System zum Einblenden von zumindest einer Information in ein Operationsfeld vorgesehen ist.

13. System gemäß einem der Ansprüche 9 bis 12, **dadurch gekennzeichnet, dass**
das System ein Objektivschutzglas (5), optional mit einer Beschichtung, aufweist,
wobei der Haltering (2) eine Führung (25) aufweist, in die das Objektivschutzglas (5) eingeführt und in einem eingeführten Zustand desselbigen gehalten werden kann, und
wobei die Führung (25) des Halterings (2) eine asymmetrische Form aufweist, sodass nur eine einzige mögliche Positionierung des Objektivschutzglases (5) in dem eingeführten Zustand desselbigen in dem Haltering (2) existiert, in der eine Ausrichtung des Objektivschutzglases (5) relativ zum Halteelement (1) festgelegt ist.

14. System gemäß Anspruch 13, **dadurch gekennzeichnet, dass** die Führung (25) des Halterings (2) und/oder das Objektivschutzglas (5) so ausgebildet ist, dass das Objektivschutzglas (5) in dem eingeführten Zustand desselbigen in dem Halteelement (1) parallel oder um einen vorbestimmten Winkel geneigt zur Schnittstelle (41) des Operationsmikroskops (4) angeordnet ist.

## Claims

1. Holding element (1) for a drape for a surgical microscope (4),
the holding element (1) having a holding ring (2) and a clearance (3) arranged in the holding ring (2) for an interface (41) of the surgical microscope (4), and
the holding ring (2) having a contour (21), delimiting the clearance (3), **characterized in that**
the contour (21) is formed so as to be not symmetrical in terms of turning with respect to an axis running parallel to a fastening direction (Y) of the holding element (1) in at least one portion of the contour (21), wherein the portion is delimited by an internal angle (α1) of at least 90°, so that, in a state in which the holding element (1) is fastened to the surgical microscope (4), there is a single possible fastening position of the holding element (1) at the interface (41) of the surgical microscope (4) corresponding to the contour (21) of the holding ring (2) in which an alignment of the holding element (1) in relation to the surgical microscope (4) is fixed, and the holding ring (2) has a structure (22) that is widened at a predetermined angle in the fastening direction (Y) of the holding element (1).

2. Holding element (1) according to Claim 1, **characterized in that** the holding ring (2) has a rubber coating on its inner surface (22) facing towards the clearance (3) for the interface (41) of the surgical microscope (4), so that the holding element (1) can be fastened by static friction at the interface (41) of the surgical microscope (4) corresponding to the contour (21) of the holding ring (2) in the single possible fastening position of the holding element (1).

3. Holding element (1) according to Claim 1 or 2, **characterized in that** the holding element (1) has a magnetic and/or magnetizable material (24) for fastening the holding element (1) to the surgical microscope (4).

4. Holding element (1) according to one of the preceding claims, **characterized in that** the holding ring (2) has a guide (25), into which an objective protection glass (5) can be inserted and held in the inserted state.

5. Holding element (1) according to Claim 4, **characterized in that** the guide (25) has an asymmetrical form, so that there is only a single possible positioning of the objective protection glass (5) in the inserted state of the same in the holding ring (2), a positioning in which an alignment of the objective protection glass (5) in relation to the holding element (1) is fixed.

6. Holding element (1) according to one of the preceding claims, **characterized in that** the asymmetrical contour (21), delimiting the clearance (3), of the holding ring (2) has at least one circular portion (26).

7. Holding element (1) according to one of the preceding claims, **characterized in that** the asymmetrical contour (21), delimiting the clearance (3), of the holding ring (2) has a groove (27).

8. Drape with a flexible portion and a holding element (1) according to one of the preceding claims, connected to the flexible portion.

9. System comprising a holding element (1) for a drape and a surgical microscope (4),
the holding element (1) having a holding ring (2) and a clearance (3), arranged in the holding ring (2), for an interface (41) of the surgical microscope (4), and
the surgical microscope (4) having the interface (41) for fastening the holding element (1) to the surgical microscope (4),
**characterized in that**
the holding ring (2) has an asymmetrical contour (21), delimiting the clearance (3), and
the interface (41) has an asymmetrical contour (42) which outwardly delimits the interface (41) and which, in a state in which the holding element (1) is fastened to the surgical microscope (4), is in contact with the asymmetrical contour (21), delimiting the clearance (3), of the holding ring (2),
the asymmetrical contour (21), delimiting the clearance (3), corresponding to the asymmetrical contour (42), outwardly delimiting the interface (41), in such a way that, in the state in which the holding element (1) is fastened to the surgical microscope (4), there is a single possible fastening position of the holding element (1) at the interface (41) in which an alignment of the holding element (1) in relation to the surgical microscope (4) is fixed.

10. System according to Claim 9, **characterized in that** the system comprises the holding element (1) according to one of Claims 1 to 7, the holding element (1) being optionally connected to a flexible portion.

11. System according to Claim 9 or 10, **characterized in that**
the interface (41) of the surgical microscope (4) has at least two optical inputs and/or outputs (43), and
the clearance (3), arranged in the holding ring (2), for the interface (41) of the surgical microscope (4) is dimensioned in such a way that the at least two optical inputs and/or outputs (43) of the surgical microscope (4) are arranged within the clearance (3) in the state in which the holding element (1) is fastened to the surgical microscope (4).

12. System according to Claim 11, **characterized in that**
one of the at least two optical inputs and/or outputs (43) is intended for an optical unit (44) of the surgical microscope (4), and
the second of the at least two optical inputs and/or outputs (43) is intended for an illumination element (46), which is optionally configured to emit light at a predetermined wavelength, a camera system (45) and/or a system for superimposing at least one item of information into an operating area.

13. System according to one of Claims 9 to 12, **characterized in that**
the system comprises an objective protection glass (5), optionally with a coating,
the holding ring (2) having a guide (25) into which the objective protection glass (5) can be inserted and held in an inserted state of the same, and
the guide (25) of the holding ring (2) having an asymmetrical form, so that there is only a single possible positioning of the objective protection glass (5) in the inserted state of the same in the holding ring (2), a positioning in which an alignment of the objective protection glass (5) in relation to the holding element (1) is fixed.

14. System according to Claim 13, **characterized in that** the guide (25) of the holding ring (2) and/or the objective protection glass (4) is/are formed in such a way that the objective protection glass (5) in the inserted state of the same in the holding element (1) is arranged parallel to or inclined at a predetermined angle in relation to the interface (41) of the surgical microscope (4).

## Revendications

1. Élément de retenue (1) destiné à un champ opératoire destiné à un microscope chirurgical (4),
l'élément de retenue (1) comportant un anneau de retenue (2) et un évidement (3) disposé dans l'anneau de retenue (2) et destiné à une interface (41) du microscope chirurgical (4), et
l'anneau de retenue (2) comportant un contour (21) qui délimite l'évidement (3),
**caractérisé en ce que**
le contour (21) est conçu, dans au moins une portion du contour (21), sans symétrie de rotation par rapport à un axe s'étendant parallèlement à une direction de fixation (Y) de l'élément de retenue (1), la portion étant délimitée par un angle intérieur (α1) d'au moins 90° de sorte que, dans un état dans lequel l'élément de retenue (1) est fixé au microscope chirurgical (4), il existe une seule position de fixation possible de l'élément de retenue (1) à l'interface (41) qui correspond au contour (21) de l'anneau de retenue (2) du microscope chirurgical (4) et dans laquelle une orientation de l'élément de retenue (1) est définie par rapport au microscope chirurgical (4), et
l'anneau de retenue (2) présente une structure (22) qui est élargie suivant un angle prédéterminé dans la direction de fixation (Y) de l'élément de retenue (1).

2. Élément de retenue (1) selon la revendication 1, **caractérisé en ce que** l'anneau de retenue (2) comporte un revêtement en caoutchouc sur sa surface intérieure (22) dirigée vers l'évidement (3) destiné à l'interface (41) du microscope chirurgical (4) de sorte que l'élément de retenue (1) puisse être fixé par friction statique dans la seule position de fixation possible de l'élément de retenue (1) à l'interface (41) du microscope chirurgical (4) qui correspond au contour (21) de l'anneau de retenue (2) .

3. Élément de retenue (1) selon la revendication 1 ou 2, **caractérisé en ce que** l'élément de retenue (1) comporte un matériau magnétique et/ou magnétisable (24) destiné à fixer l'élément de retenue (1) au microscope chirurgical (4).

4. Élément de retenue (1) selon l'une des revendications précédentes, **caractérisé en ce que** l'anneau de retenue (2) comporte un guide (25) dans lequel un verre de protection d'objectif (5) peut être inséré et maintenu à l'état inséré.

5. Élément de retenue (1) selon la revendication 4, **caractérisé en ce que** le guide (25) a une forme asymétrique de sorte qu'il n'existe qu'une seule position possible du verre de protection d'objectif (5), lorsque celui-ci est inséré dans l'anneau de retenue (2), dans laquelle une orientation du verre de protection d'objectif (5) est définie par rapport à l'élément de retenue (1).

6. Élément de retenue (1) selon l'une des revendications précédentes, **caractérisé en ce que** le contour asymétrique (21) de l'anneau de retenue (2), lequel contour délimite l'évidement (3), comporte au moins une portion circulaire (26).

7. Élément de retenue (1) selon l'une des revendications précédentes, **caractérisé en ce que** le contour asymétrique (21) de l'anneau de retenue (2), lequel contour délimite l'évidement (3), comporte une rainure (27).

8. Champ opératoire comprenant une portion flexible et un élément de retenue (1) selon l'une des revendications précédentes qui est relié à la portion flexible.

9. Système comportant un élément de retenue (1) destiné à un champ opératoire et un microscope chirurgical (4),
l'élément de retenue (1) comportant un anneau de retenue (2) et un évidement (3) disposé dans l'anneau de retenue (2) et destiné à une interface (41) du microscope chirurgical (4), et
le microscope chirurgical (4) comportant l'interface (41) destinée à fixer l'élément de retenue (1) au microscope chirurgical (4),
**caractérisé en ce que**
l'anneau de retenue (2) présente un contour asymétrique (21) qui délimite l'évidement (3), et
l'interface (41) comporte un contour asymétrique (42) qui délimite l'interface (41) par rapport à l'extérieur et qui est en contact avec le contour asymétrique (21) de l'anneau de retenue (2), lequel contour délimite l'évidement (3), dans un état dans lequel l'élément de retenue (1) est fixé au microscope chirurgical (4),
le contour asymétrique (21) qui délimite l'évidement (3) correspondant au contour asymétrique (42) qui délimite l'interface (41) par rapport à l'extérieur **en ce que**, dans l'état dans lequel l'élément de retenue (1) est fixé au microscope chirurgical (4), il existe une seule position de fixation possible de l'élément de retenue (1) à l'interface (41) dans laquelle une orientation de l'élément de retenue (1) par rapport au microscope chirurgical (4) est déterminée.

10. Système selon la revendication 9, **caractérisé en ce que** le système comporte l'élément de retenue (1) selon l'une des revendications 1 à 7, l'élément de retenue (1) étant éventuellement relié à une portion flexible.

11. Système selon la revendication 9 ou 10, **caractérisé en ce que**
l'interface (41) du microscope chirurgical (4) comporte au moins deux entrées et/ou sorties optiques (43), et
l'évidement (3) disposé dans l'anneau de retenue (2) et destiné à l'interface (41) du microscope chirurgical (4) a des dimensions telles que les au moins deux entrées et/ou sorties optiques (43) du microscope chirurgical (4) sont disposées à l'intérieur de l'évidement (3) dans l'état dans lequel l'élément de retenue (1) est fixé au microscope chirurgical (4).

12. Système selon la revendication 11, **caractérisé en ce que**
une des au moins deux entrées et/ou sorties optiques (43) est prévue pour une optique (44) du microscope chirurgical (4), et
la deuxième des au moins deux entrées et/ou sorties optiques (43) est prévue pour un élément d'éclairage (46) qui est éventuellement conçu pour émettre de la lumière dans une longueur d'onde prédéterminée, un système de caméra (45) et/ou un système d'affichage d'au moins une information dans une zone opérationnelle.

13. Système selon l'une des revendications 9 à 12, **caractérisé en ce que** le système comporte un verre de protection d'objectif (5), éventuellement pourvu d'un revêtement, l'anneau de retenue (2) comportant un guide (25) dans lequel le verre de protection d'objectif (5) peut être inséré et maintenu dans un état inséré de celui-ci, et
le guide (25) de l'anneau de retenue (2) ayant une forme asymétrique de sorte qu'il n'existe qu'une seule position possible du verre de protection d'objectif (5), lorsque celui-ci est inséré dans l'anneau de retenue (2), dans laquelle une orientation du verre de protection d'objectif (5) par rapport à l'élément de retenue (1) est définie.

14. Système selon la revendication 13, **caractérisé en ce que** le guide (25) de l'anneau de retenue (2) et/ou le verre de protection d'objectif (5) est conçu de telle sorte que le verre de protection d'objectif (5), lorsque celui-ci est inséré dans l'élément de retenue (1), est disposé parallèlement à l'interface (41) du microscope chirurgical (4) ou incliné suivant un angle prédéterminé par rapport à celle-ci.
